(19) Europäisches Patentamt
European Patent Office
Office européen des brevets

(11) Publication number: 0 054 366 A2

(12) **EUROPEAN PATENT APPLICATION**

(21) Application number: 81305572.0

(22) Date of filing: 25.11.81

(51) Int. Cl.³: C 07 C 43/315
C 11 D 1/72

(30) Priority: 15.12.80 GB 8040065

(43) Date of publication of application: 23.06.82 Bulletin 82/25

(84) Designated Contracting States: AT BE CH DE FR GB IT LI NL SE

(71) Applicant: IMPERIAL CHEMICAL INDUSTRIES PLC
Imperial Chemical House Millbank
London SW1P 3JF(GB)

(72) Inventor: Hancock, Roger Ian
9 Bentinck Road
Fairfield Stockton-on-Tees Cleveland(GB)

(72) Inventor: Greenshields, James Nairn
27 Fernwood
Marple Bridge Stockport Cheshire(GB)

(74) Representative: Locke, Timothy John et al,
Imperial Chemical Industries PLC Legal Department: Patents Thames House North Millbank
London SW1P 4QG(GB)

(54) **Novel surface active agents and compositions comprising them.**

(57) Novel surface active agents are acetals of $C_{11}$ to $C_{19}$ aldehydes with alkoxylated lower alcohols. They show good performance in built liquid low foaming compositions.

EP 0 054 366 A2

Novel Surface Active Agents and Compositions Comprising Them

THIS INVENTION relates to novel surface active agents and compositions comprising them.

A number of surface active acetals obtained by condensing alkyl monoethers of polyethylene glycols with aliphatic aldehydes have been investiaged by B Burczyk and A Sokolowski, Tenside Detergents 15 (1978) page 2. They show certain materials of this type were likely to be easily biodegradable and have low foaming properties. The materials which they investigated had a total of hydrophobic carbon atoms (by which is meant the sum of the carbon atoms in the aldehyde and both alkyl groups) in the range 3 to 8.

Unfortunately the performance of acetals of this type in practical detergent compositions which include builders is poor.

We have found that a novel group of acetals shows good performance in practical detergent compositions containing builders.

The novel compounds of this invention have the formula

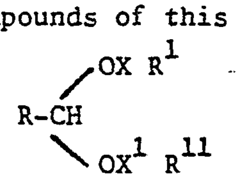

where R is an alkyl group having 11 to 19 carbon atoms and $R^1$ and $R^{11}$ are individually alkyl groups having 1 to 4 carbon atoms. The groups X and $X^1$ are individually alkylene oxide or polyalkylene oxide residues having 1 to 15 alkylene oxide residues in each. These may be ethylene oxide or propylene oxide residues or both as random or block copolymers.

It is preferred that the total number of carbon atoms in the groups R, $R^1$ and $R^{11}$ should be in the range 15 to 22 and that the total carbon atom content of the molecule should be in the range 32 to 60 carbon atoms.

The compounds according to the invention may be made by reacting a monoalkyl ether of a glycol or polyglycol with an aliphatic aldehyde in the presence of an acid catalyst, for example phosphoric acid, hydrochloric acid or p-toluenesulphonic acid in a concentration of for example 0.1 to 2% and preferably 0.5 to 1% by weight. The temperature is suitably 5 to 200°C for example 60 to

150°C. Water is liberated in the course of the reaction and may be removed by operating under vacuum and distilling the water off or by operating in the presence of an immiscible azeotroping agent for example toluene, cyclohexane or a xylene. The reactants are suitably in stoichimetric proportions though the glycol ether may be present in up to a 50% excess if desired.

Detergent compositions comprising the novel compounds suitably include a builder for example a phosphate for example sodium tripolyphosphate, nitrilotriacetic acid or a zeolite.

EXAMPLE

Compounds according to the invention were prepared as follows:

A mixed $C_{13}$ to $C_{15}$ aldehyde (67% by weight $C_{13}$ and 33% by weight $C_{15}$) consisting of 50% straight chain aldehydes and 50% 2-methyl branched aldehydes (850 grams) was added with 1,200 grams methanol and 0.5 grams of concentrated hydrochloric acid (specific gravity 1.18) to a 3 litre vessel and maintained at 45 to 55°C for 15 hours, at the end of which time the reaction was judged to have reached completion based on the reduction in intensity of the carbonyl peak as monitored by infra-red spectroscopy.

$Ca(OH)_2$ (10 grams) was added to neutralise the hydrochloric acid. Excess methanol and water were then removed by distillation and the product was filtered. The product has an estimated aldehyde content of 1.5% by weight, a water content of 0.3% by weight and a methanol content of 0.5% by volume. The product consisted essentially of the dimethyl acetal of the mixed aldehyde.

Some of this dimethyl acetal (387 grams) and the product of condensing 1 mole of methanol with 6 moles of ethylene oxide (873 grams) were charged into a 2 litre flask fitted with a side arm condenser and stirred under an atmosphere of nitrogen. Hydrochloric acid (8 grams) (specific gravity of 1.18) was added dropwise and the flask was heated to 100-150°C until no more methanol distillate was collected. The product was neutralised with $Ca(OH)_2$ (30 grams) to give a pH after neutralisation of 8.5. The product was filtered through keiselguhr to give a clear yellowy orange liquid of formula

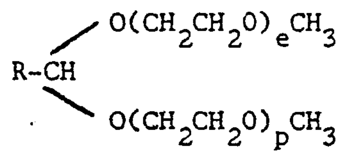

where R is an alkyl group derived from the mixed aldehyde and e and p average 6. It is referred to hereinafter as "product 1".

A further product according to the invention was made as follows:

The dimethyl acetal of the mixed aldehyde (258 grams) and the product of condensing 1 mole of methanol with 10 moles of ethylene oxide (912.2 grams) was fed into a flask and stirred and concentrated hydrochloric acid (5 grams, specific gravity of 1.18) was added dropwise. The flask was heated with stirring under nitrogen to remove methanol of reaction. After 5 hours at a flask temperature of 100 to 150$^{o}$C the stoichiometric amount of methanol had been collected.

The product was a homogeneous orangey yellow liquid. A 10% solution in normal hexane was shown by gas liquid chromatography to be free from aldehyde and the dimethyl acetal. The weight of the crude product was 1080 grams. $Ca(OH)_2$ (20 grams) was added to the crude product which was stirred under an atmosphere of nitrogen for 2 hours. The neutralised product was filtered through keiselguhr to give a clear yellow/orange liquid which was of formula

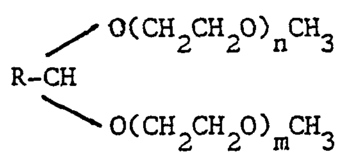

where R is derived from the mixed aldehyde and m and n average 10. It is referred to hereinafter as product 2.

Product 1, product 2 and a corresponding product prepared by similar means but in which e and p average 3 (product 3) were tested with comparison with standard alcohol ethyoxylates which were the condensation products of 1 mole of a mixed alcohol which was 67% by weight $C_{13}$ alcohol and 33% by weight $C_{15}$ alcohol (50% linear and 50% 2-methyl branched) with respectively 7 and 9 moles ethylene oxide (hereinafter referred to as ethyoxylate 7 and 9 respectively).

The above products were tested by placing 3 pieces of uniformly Krefeld soiled cotton or polyester cotton cloth and 1

piece of clean cotton cloth (10 by 7.5 cm rectangles) in 1 litre of water (50 parts per million hardness expressed as calcium carbonate) containing in the case of the results of Table 1, 2.5 grams of the stated surfactant and in the case of Tables 2 and 3, 5 grams of the compositions indicated in the following table, and washing at 60°C in a US Testing Company Tergotometer (model 7243). The cloths were rinsed in cold water and ironed and their reflectance before and after washing was measured in a Gardner reflectometer.

| Component | Composition type | | | | | | | | |
|---|---|---|---|---|---|---|---|---|---|
| | (TYPE A) | | | | | (TYPE B) | | | |
| | 1 | 2 | 3 | 4 | 5 | 1 | 2 | 3 | 4 |
| Ethoxylate 7 | 10 | | | | | 3 | | | |
| Ethoxylate 9 | | 10 | | | | | 3 | | |
| Product 1 | | | 10 | | | | | 3 | |
| Product 2 | | | | 10 | | | | | 3 |
| Product 3 | | | | | 10 | | | | |
| Linear dodecyl benzene sulphonic acid | - | - | - | - | - | 3 | 3 | 3 | 3 |
| Coconut monoethanolamide* | 1 | 1 | 1 | 1 | 1 | 1 | 1 | 1 | 1 |
| Tallow fatty acid | 1 | 1 | 1 | 1 | 1 | 3 | 3 | 3 | 3 |
| Sodium tripolyphosphate | 45 | 45 | 45 | 45 | 45 | 45 | 45 | 45 | 45 |
| Sodium metasilicate | 12 | 12 | 12 | 12 | 12 | 12 | 12 | 12 | 12 |
| Carboxymethyl cellulose | 0.3 | 0.3 | 0.3 | 0.3 | 0.3 | 0.3 | 0.3 | 0.3 | 0.3 |
| Optical brightener | 0.3 | 0.3 | 0.3 | 0.3 | 0.3 | 0.3 | 0.3 | 0.3 | 0.3 |
| Ethylene diamine tetraacetic acid (sodium salt) | 0.3 | 0.3 | 0.3 | 0.3 | 0.3 | 0.3 | 0.3 | 0.3 | 0.3 |
| sodium carbonate | 10.1 | 10.1 | 10.1 | 10.1 | 10.1 | 12.1 | 12.1 | 12.1 | 12 |
| sodium percarbonate | 20 | 20 | 20 | 20 | 20 | 20 | 20 | 20 | 2 |

all figures are quoted as $\%^{w}/w$

*The monoethanolamide of coconut-oil derived mixed acids.

In the following tables the figures quoted are the difference between the reflectance of the cloth before and after washing, in arbitrary units.

Krefold soil comprises the following ingredients: Stearic acid 15% $^w/w$, Oleic Acid 15% $^w/w$, Octadecanol 8% $^w/w$, Gycerol Tristearate 10% $^w/w$, Paraffin Oil 25% $^w/w$, Aracus Oil 20% $^w/w$, Cholesterol 7% $^w/w$.

Table 1

| PRODUCT | COTTON | | POLYESTER COTTON | |
|---|---|---|---|---|
| | Soiled | Redeposition | Soiled | Redeposition |
| Ethoxylate 7 | 23.13 | -0.6 | 13.4 | -0.5 |
| Ethoxylate 9 | 22.2 | -0.3 | 18.6 | +1.1 |
| Product 1 | 13.9 | -2.0 | 5.7 | -1.1 |
| Product 2 | 20.3 | -0.5 | 1.8 | -0.7 |

Table 2

Type A Formulation

| Formu-lation number | COTTON | | POLYESTER COTTON | |
|---|---|---|---|---|
| | Soiled | Redeposition | Soiled | Redeposition |
| 1 | 26.8 | -0.7 | 22.9 | +2.4 |
| 2 | 25.6 | +0.1 | 22.2 | +2.7 |
| 3 | 26.2 | +1.2 | 19.3 | +2.3 |
| 4 | 23.7 | +2.4 | 17.0 | +2.2 |
| 5 | - | - | 22.3 | +2.4 |

Table 3

Type B Formulation

| Formulation number | COTTON | | POLYESTER COTTON | |
|---|---|---|---|---|
| | Soiled | Redeposition | Soiled | Redeposition |
| 1 | 18.1 | +1.9 | 24.0 | +6.3 |
| 2 | 17.7 | +0.2 | 24.3 | +6.0 |
| 3 | 17.7 | +0.4 | 23.4 | +6.6 |
| 4 | 16.0 | -0.3 | 20.1 | +1.1 |

The above figures show that built compositions containing the compounds of the invention, show a comparable performance to conventional high performance non-ionic surfactnats. The new compositions however are low foaming liquids whereas the conventional products are solids and viscous liquids at room temperature and foam to a greater extent.

Foam heights were determined at 25°C using standard Ross Miles equipment for aqueous solutions containing 0.1%$^{w}$/w of products 1 and 2, and of product 4 which corresponds in formula to product 1 except that the values of e and p average 8. The solutions contained the amounts of calcium (as calcium chloride) shown in the table. (ppm means parts per million by weight pressed as calcium). Foam heights (in mm) are shown initially and after 5 minutes.

| Product | Foam Heights (mm) | | | | | |
|---|---|---|---|---|---|---|
| | 50 ppm Ca | | 150 ppm Ca | | 300 ppm Ca | |
| | Initial | After 5 mins | Initial | After 5 mins | Initial | After 5 mins |
| 1 | 50 | 15 | 40 | 10 | 45 | 10 |
| 2 | 45 | 5 | 85 | 15 | 85 | 15 |
| 4 | 80 | 15 | 90 | 10 | 90 | 15 |

The figures above show moderate initial foaming followed by rapid collapse of the foam. The products are thus suitable for low foaming surfactant compositions.

As indicated by the foregoing examples, compounds according to the invention may be made by reacting a monoalkyl ether of a glycol or polyglycol with an acetal of an aliphatic aldehyde with an alcohol having 1 to 4 carbon atoms in the presence of an acid catalyst, for example phosphoric acid, hydrochloric acid or p-toluene sulphonic acid in a concentration of for example 0.1 to 2% and preferably 0.5 to 1% by weight. The temperature is suitably 5 to 200°C for example 60 to 150°C. The alcohol is liberated from the acetal in the course of the reaction and may be removed by distillation during the reaction. The reactants are suitably in stoichiometric proportions though the glycol ether may be present in up to a 50% excess if desired.

Claims

1. Acetals of formula

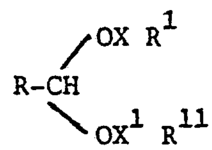

where R is an alkyl group having 11 to 19 carbon atoms and $R^1$ and $R^{11}$ are individually alkyl groups having 1 to 4 carbon atoms and in which the groups X and $X^1$ are individually alkylene oxide or polyalkylene oxide residues having 1 to 15 alkylene oxide residues in each.

2. Acetals as claimed in claim 1 in which the groups X and $X^1$ are polyethylene oxide residues.

3. Acetals as claimed in claim 1 or 2 in which the total number of carbon atoms in the groups R, $R^1$ and $R^{11}$ is in the range 15 to 22 and in which the total carbon atom content of the molecule is in the range 32 to 60 carbon atoms.

4. A process for the production of acetals as claimed in any preceding claim in which a monoalkylether of a glycol or polyglycol is reacted with an aliphatic aldehyde or an acetal thereof with a $C_1$ to $C_4$ alcohol in the presence of an acid catalyst.

5. A process as claimed in claim 4 in which the catalyst is phosphoric acid, hydrochloric acid or paratoluenesulphonic acid.

6. A process as claimed in claim 4 or 5 which is carried out at a temperature of 60 to 150°C.

7. A process as claimed in claim 4, 5 or 6 in which the water or alcohol liberated in the course of the reaction is removed by distillation during the reaction.

8. A surfactant composition which comprises an acetal as claimed in claim 1, 2 or 3 and a builder.

9. A composition as claimed in claim 8 which comprises a phosphate builder.